(19) Europäisches Patentamt
European Patent Office
Office européen des brevets

(11) Veröffentlichungsnummer: **0 422 537 B1**

(12) **EUROPÄISCHE PATENTSCHRIFT**

(45) Veröffentlichungstag der Patentschrift: **11.05.94**

(51) Int. Cl.⁵: **C07C 255/46**, A01N 37/34, C07C 255/61, C07C 255/64

(21) Anmeldenummer: **90119208.8**

(22) Anmeldetag: **06.10.90**

Die Akte enthält technische Angaben, die nach dem Eingang der Anmeldung eingereicht wurden und die nicht in dieser Patentschrift enthalten sind.

# (54) Cyclohexenonverbindungen, Verfahren zu ihrer Herstellung sowie ihre Verwendung als Herbizide oder das Pflanzenwachstum regulierende Mittel.

(30) Priorität: **13.10.89 DE 3934204**

(43) Veröffentlichungstag der Anmeldung:
**17.04.91 Patentblatt 91/16**

(45) Bekanntmachung des Hinweises auf die Patenterteilung:
**11.05.94 Patentblatt 94/19**

(84) Benannte Vertragsstaaten:
**AT BE CH DE ES FR GB IT LI NL**

(56) Entgegenhaltungen:
**DE-A- 2 439 104**
**DE-A- 3 440 410**
**DE-A- 3 523 862**

(73) Patentinhaber: **BASF Aktiengesellschaft**
**Carl-Bosch-Strasse 38**
**D-67063 Ludwigshafen(DE)**

(72) Erfinder: **Kast, Jürgen, Dr.**
**Kastanienstrasse 24**
**W-6737 Boehl-Iggelheim(DE)**
Erfinder: **Meyer, Norbert, Dr.**
**Dossenheimer Weg 22**
**W-6802 Ladenburg(DE)**
Erfinder: **Misslitz, Ulf, Dr.**
**Am Herzl 40**
**W-6730 Neustadt(DE)**
Erfinder: **Schubert, Jürgen, Dr.**
**Am Oberen Luisenpark 2**
**W-6800 Mannheim 1(DE)**
Erfinder: **Jung, Johann, Prof.-Dr.**
**Hardenburgstrasse 19**
**W-6703 Limburgerhof(DE)**
Erfinder: **Rademacher, Wilhelm, Dr.**
**Austrasse 1**
**W-6703 Limburgerhof(DE)**
Erfinder: **Westphalen, Karl-Otto, Dr.**
**Mausbergweg 58**
**W-6720 Speyer(DE)**
Erfinder: **Wuerzer, Bruno, Dr.**
**Ruedigerstrasse 13**
**W-6701 Otterstadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

**Beschreibung**

Die vorliegende Erfindung betrifft Cyclohexenonverbindungen der Formel I

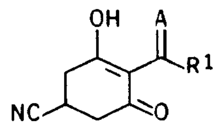

I,

in der die Substituenten die folgende Bedeutung haben:
$R^1$ $C_1$-$C_{20}$-Alkyl, $C_2$-$C_{20}$-Alkenyl, $C_2$-$C_{20}$-Alkinyl, $C_3$-$C_6$-Cycloalkyl, jeweils ggf. substituiert durch einen oder mehrere Halogensubstituenten, $C_2$-$C_6$-Alkoxyalkyl, $C_2$-$C_6$-Alkylthioalkyl, Benzyl oder Phenyl, wobei die aromatischen Kerne jeweils unsubstituiert oder ein- bis dreifach durch Halogen, Cyano, $C_1$-$C_4$-Alkyl, $C_1$-$C_4$-Alkoxy, $C_1$-$C_4$-Halogenalkyl, oder Nitro substituiert sind;
A Sauerstoff, $NOR^2$ oder $NR^3$, wobei
$R^2$ $C_1$-$C_4$-Alkyl, $C_3$-$C_4$-Alkenyl, $C_3$-$C_4$-Alkinyl, jeweils gegebenenfalls substituiert mit einem oder mehreren Substituenten aus der Gruppe Halogen, $C_1$-$C_4$-Alkoxy, Phenyl bzw. Heteroaryl mit 5 bis 6 Ringgliedern und 1 bis 2 Heteroatomen wie Stickstoff, Sauerstoff oder Schwefel, wobei die beiden letztgenannten Reste unsubstituiert oder ein bis dreifach durch Halogen, $C_1$-$C_4$-Alkyl, $C_1$-$C_4$-Alkoxy, $C_1$-$C_4$-Halogenalkyl oder Nitro substituiert sind,
$R^3$ Wasserstoff, $C_1$-$C_6$-Alkyl, $C_1$-$C_4$-Hydroxyalkyl, $C_2$-$C_4$-Alkoxyalkyl, $C_2$-$C_4$-Alkylthioalkyl, Phenyl oder Benzyl, wobei die beiden letztgenannten Reste unsubstituiert oder ein- bis dreifach durch Halogen, $C_1$-$C_4$-Alkyl, $C_1$-$C_4$-Alkoxy, $C_1$-$C_4$-Halogenalkyl oder Nitro substituiert sind,
sowie deren landwirtschaftlich brauchbaren Salze und Veresterungsprodukte mit Carbonsäuren, Sulfon- oder Phosphonsäuren.

Außerdem betrifft die Erfindung Verfahren zur Herstellung der Verbindungen I und herbizide sowie des Pflanzenwachstum regulierende Mittel, welche mindestens eine Cyclohexenonverbindung der Formel I enthalten.

Aus der DE-A 24 39 104 geht hervor, daß Cyclohexenonoximether mit einer Cyanogruppe in 4-Position und gleichzeitig einer Dimethylsubstitution in 5-Position wie 2-[1-(Ethoxyamino)ethyliden]-4-cyano-5,5-dimethylcyclohexan-1,3-dion oder 2-[1-(Allyloxyamino)butyliden]-4-Cyano-5,5-dimethylcyclohexan-1,3-dion eine gute herbizide Wirkung aufweisen.

Gemäß der DE-A 35 23 862 werden u.a. Cyclohexenonverbindungen der Struktur I'

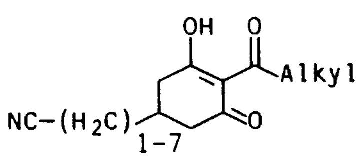

I'

als Mittel zur Regulierung des Pflanzenwachstums offenbart. Oximether dieser Struktur sind in DE-A 34 40 410 als herbizid wirksame Substanzen beschrieben.

Der Erfindung lag nun die Aufgabe zugrunde, neue herbizid und/oder bioregulatorisch wirksame Substanzen zu finden.

Demgemäß werden die eingangs definierten Cyclohexenonverbindungen gefunden. Verbindungen I, in denen A für den Rest $NOR^2$ steht, zeigen sehr gute herbizide Eigenschaften z.B. gegen Arten aus der Familie der Gräser (Gramineen), während solche, in denen A Sauerstoff oder eine Iminogruppe $NR^3$ bedeuten, vorteilhaft zur Regulierung des Pflanzenwachstums, insbesondere zur Halmverkürzung eingesetzt werden können.

Im Hinblick auf die bestimmungsgemäße Verwendung der Verbindungen I kommen als Substituenten bevorzugt folgende Reste in Betracht:

$R^1$

- $C_1$- bis $C_{20}$-, insbesondere $C_1$- bis $C_6$-Alkyl wie Methyl, Ethyl, Propyl, Isopropyl, n-Butyl, iso-Butyl, s-Butyl, t-Butyl, n-Pentyl und n-Hexyl
- $C_2$- bis $C_6$-Alkenyl wie z.B. Vinyl, Allyl, But-2-enyl, But-3-enyl, Pent-2-enyl, Pent-3-enyl, Pent-4-enyl, Hex-2-enyl, Hex-3-enyl, Hex-4-enyl, Hex-5-enyl

- $C_2$- bis $C_6$-Alkinyl wie z.B. Ethinyl, Propargyl, But-2-inyl, But-3-inyl, Pent-2-inyl, Pent-3-inyl, Pent-4-inyl, Hex-2-inyl
- $C_3$- bis $C_6$-Cycloalkyl wie Cyclopropyl, Cyclobutyl, Cyclopentyl, Cyclohexyl

wobei die genannten Alkyl-, Alkenyl-, Alkinyl- und Cycloalkylreste ggf. durch ein oder mehrere, z.B. 1 bis 4 Halogenatome wie Fluor, Chlor oder Brom substituiert sind,

- $C_2$- bis $C_6$-Alkoxyalkyl, z.B. $C_1$-$C_3$-Alkoxy-$C_1$-$C_4$-Alkyl wie Methoxymethyl, Ethoxymethyl, Propyloxymethyl, 1-Methoxyethyl, 2-Methoxyethyl, 1-Methoxyethyl, 2-Ethoxyethyl, 2-Propyloxyethyl, 1-Methoxypropyl, 2-Methoxypropyl, 3-Methoxypropyl, 1-Ethoxypropyl, 2-Ethoxypropyl, 3-Ethoxypropyl, 1-Methoxybutyl, 2-Methoxybutyl, 3-Methoxybutyl, 4-Methoxybutyl, 1-Ethoxybutyl, 2-Ethoxybutyl, 3-Ethoxybutyl, 4-Ethoxybutyl
- $C_2$- bis $C_6$-Alkylthioalkyl, z.B. Methylthiomethyl, Ethylthiomethyl, Propylthiomethyl, Methylthioethyl, Ethylthioethyl, Propylthioethyl, Methylthiopropyl, Ethylthiopropyl
- Benzyl oder Phenyl, wobei die aromatischen Kerne ggf. ein- bis dreifach durch Halogen wie Chlor, Brom, Fluor, durch Cyano, Nitro, $C_1$-$C_4$-Alkyl wie Methyl, Ethyl, Propyl oder Butyl, $C_1$-$C_4$-Alkoxy wie Methoxy, Ethoxy, Propoxy oder Butoxy, $C_1$-$C_4$-Halogenalkyl wie Trifluormethyl, Difluorchlormethyl, Difluormethyl, Trichlormethyl, Pentafluorethyl, Tetrafluorethyl, Fluorpropyl, Fluorbutyl, Chlorpropyl, Chlorbutyl substiutiert sind.

$R^2$

- ggf. ein- oder mehrfach, z.B. 1 bis 3fach substituiertes $C_1$- bis $C_4$-Alkyl wie Methyl, Ethyl, Propyl, iso-Propyl, n-Butyl, iso-Butyl, sec.-Butyl, t-Butyl
- ggf. ein- bis 3fach substituiertes $C_3$- oder $C_4$-Alkenyl wie Allyl, But-2-enyl oder But-3-enyl
- ggf. einfach substituiertes $C_3$- oder $C_4$-Alkinyl wie Propargyl, But-2-inyl oder Butin-3-yl

Als Substituenten kommen in Betracht:

- Halogen wie Fluor, Chlor oder Brom,
- $C_1$- bis $C_4$-Alkoxy wie für $R^1$ genannt,
- Phenyl oder Heteroaryl mit 5 bis 6 Ringgliedern und 1 bis 2 Heteroatomen wie Stickstoff, Sauerstoff oder Schwefel, z.B. Pyrazolyl, Imidazolyl, Isoxazolyl, Oxazolyl, Isothiazolyl, Thioxazolyl, Pyridyl, Pyrimidyl, Furyl, Thienyl, Pyrrolyl, wobei Phenyl und Thienyl bevorzugt sind. Die genannten aromatische Reste können ein- bis dreifach durch Halogen, $C_1$-$C_4$-Alkyl, $C_1$-$C_4$-Alkoxy oder $C_1$-$C_4$-Halogenalkyl substituiert sein. Beispielsweise seien Phenyl, Methylphenyl, Ethylphenyl, Propylphenyl, Butylphenyl, Methoxyphenyl, Ethoxyphenyl, Propyloxyphenyl, Butyloxyphenyl, 3,4-Dichlorphenyl, 2,6-Dichlorphenyl, 4-Chlorphenyl, 4-Bromphenyl, 2-Bromphenyl, 3-Trifluormethylphenyl, Thien-2-yl, 5-Chlorthienyl, 5-Chlorthien-2-yl, 4-Bromthien-2-yl, Thien-3-yl, 5-Chlorthien-3-yl, 4,5-Dichlorthien-3-yl, 5-Bromthien-3-yl, 2,5-Dichlorthien-3-yl, Isoxazolidin-5-yl, Oxazolyl, Isoxazolinyl, Thiazolyl, Thiazolinyl, Thiazolidinyl, 3-Methylisoxazol-5-yl, 3-Methoxymethylisoxazol-5-yl, 3-Phenylisoxazol-5-yl, 5-i-Propyl-1,3,4-oxadiazol-2-yl genannt.

$R^3$

- Wasserstoff,
- $C_1$- bis $C_6$-Alkyl wie für $R^1$ genannt,
- $C_1$- bis $C_4$-Hydroxyalkyl wie 2-Hydroxyethyl, 2-Hydroxypropyl, 2-Hydroxybutyl, 3-Hydroxypropyl, 3-Hydroxybutyl
- $C_1$- bis $C_4$-Alkoxyalkyl wie 2-Methoxyethyl, 2-Ethoxyethyl, 2-Methoxypropyl, 3-Methoxypropyl
- $C_1$- bis $C_4$-Alkylthioalkyl wie 2-Methylthioethyl, 2-Ethylthioethyl, 2-Methylthiopropyl, 3-Methylthiopropyl
- Phenyl oder Benzyl, wobei die aromatischen Reste ein- bis dreifach durch Halogen, $C_1$-$C_4$-Alkyl, $C_1$-$C_4$-Halogenalkyl oder Nitro substituiert, im einzelnen wie im Fall von $R^1$ genannt, substituiert sein können.

Besonders bevorzugt sind in Formel I folgende Substituenten:

$R^1$ in Formel I bedeutet Methyl, Ethyl, n-Propyl, i-Propyl, n-Butyl, i-Butyl, s-Butyl, t-Butyl, n-Pentyl, Cyclopropyl, Cyclobutyl, Cyclopentyl, Cyclohexyl;

$R^2$, im Fall, daß A für $NOR^2$ steht, bedeutet Ethyl, Propyl, Allyl, (E)-But-2-en-1-yl, Propargyl, But-2-in-1-yl, 2-Fluorethyl, (E)-3-Chlorprop-2-en-1-yl, 4-Phenylbut-2-en-1-yl, 4-(4-Fluorphenyl)-but-2-en-1-yl, 4-(4-Chlorphenyl)-but-2-en-1-yl, 4-(4-Fluorphenyl)-but-3-en-1-yl, 4-(4-Chlorphenyl)-but-3-en-1-yl, 5-Chlorthenyl;

$R^3$, im Fall, daß A für $NR^3$ steht, bedeutet Wasserstoff, Methyl, Ethyl, n-Propyl, i-Propyl, n-Butyl, s-Butyl, t-Butyl, n-Pentyl, n-Hexyl, Allyl, Hydroxyethyl, 2-Methoxyethyl, Benzyl, Phenyl.

Von den genannten bevorzugten Substituenten sind folgende Reste ganz besonders bevorzugt:

$R^1$ Methyl, Ethyl, n-Propyl, n-Butyl, Cyclopropyl;

$R^2$ (A = $NOR^2$) Ethyl, Allyl, (E)-But-2-en-1-yl, Propargyl, But-2-in-1-yl, (E)-3-Chlorprop-2-en-1-yl, 4-(4-Fluorphenyl)-but-2-en-1-yl, 4-(4-Chlorphenyl)-but-2-en-1-yl, 4-(4-Fluorphenyl)-but-3-en-1-yl, 4-(4-Chlorphenyl)-but-3-en-1-yl, 5-Chlorthenyl;

$R^3$ (A = $NR^3$) Wasserstoff, Methyl, Ethyl, n-Propyl, i-Propyl, n-Butyl, s-Butyl, i-Butyl, n-Hexyl, Allyl, 2-Methoxyethyl, Benzyl, Phenyl.

Als Salze der Verbindungen der Formel I kommen landwirtschaftlich brauchbare Salze, beispielsweise Alkalimetallsalze, insbesondere das Natrium- oder Kaliumsalz, Erdalkalisalze insbesondere das Calcium-, Magnesium- oder Bariumsalz, Mangan-, Kupfer-, Zink- oder Eisensalz sowie Ammonium-, Phosphonium, Sulfonium- oder Sulfoxoniumsalze, beispielsweise Ammoniumsalze, Tetraalkylammoniumsalze, Benzyltrialkylammoniumsalze, Triaikylsulfoniumsalze oder Trialkylsulfoniumsalze in Betracht.

Als Veresterungsprodukte kommen z.B. Ester mit niedermolekularen Carbonsäuren z.B. $C_1$-$C_6$-Carbonsäuren wie Essigsäureester, Propionsäureester sowie Benzoesäureester oder Benzolsulfonsäureester in Betracht.

Die Herstellung der Verbindungen I mit A = $NOR^2$ oder $NR^3$ kann vorteilhaft über das zentrale Zwischenprodukt Ia (Verbindung I mit A = Sauerstoff) erfolgen.

Die Verbindung Ia wird erfindungsgemäß durch Umsetzung der entsprechenden formylsubstituierten Cyclohexenonverbindung II

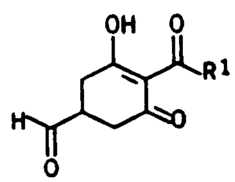

II

mit Hydroxylamin-O-sulfonsäure in einem inerten Lösungsmittel bei Temperaturen von 0 bis 150 °C hergestellt.

Als besonders günstig hat es sich erwiesen, die Umsetzung in Wasser mit 1 bis 100 Gew.-Teilen Wasser, bezogen auf Ausgangsstoff II, bei einem pH von 1 bis 9 und einer Temperatur von 20 bis 80 °C, insbesondere 20 bis 40 °C vorzunehmen.

Der Reaktionsweg ist im folgenden Schema wiedergegeben:

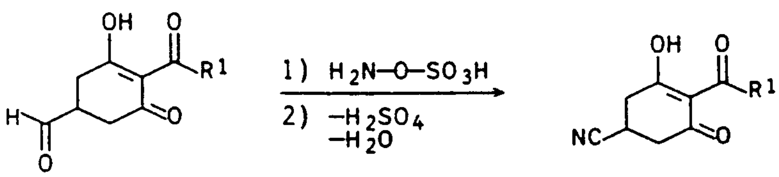


II Ia

Die Formylverbindung II ist wie in EP-A 233 568 beschrieben zugänglich.

Die Umsetzung kann in homogener oder heterogener wäßriger Phase, ggf. unter Zusatz eines Puffers durchgeführt werden. Dazu wird die Formylverbindung II in Wasser suspendiert oder durch Zusatz eines wassermischbaren organischen Lösungsmittels wie z.B. Methanol oder Ethanol in Lösung gebracht. Es ist auch möglich, die Formylverbindung II in wassernichtmischbaren Lösungsmitteln wie Ethern, z.B. Diethylether, Tetrahydrofuran; chlorierten Kohlenwasserstoffen wie Methylenchlorid, Chloroform, Dichlorethan; Estern wie Essigester oder aromatischen Verbindungen wie Benzol, Toluol, Xylole zu lösen. Zu der Mischung aus Lösungsmittel und Ausgangsstoff II gibt man die Hydroxylamin-O-sulfonsäure in fester Form oder als wäßrige Lösung.

Wenn die Umsetzung in heterogener Phase vorgenommen wird, ist es vorteilhaft für eine intensive Durchmischung der Phasen zu sorgen.

Um eine vollständige Umsetzung zur Cyanoverbindung zu erreichen, empfiehlt es sich die Reaktionskomponenten in äquimolaren Mengen einzusetzen. Aus verfahrenstechnischen Gründen kann es ratsam sein die Formylverbindung II oder die Hydroxylamin-O-sulfonsäure im Überschuß z.B. von 1 bis 100 Mol-% einzusetzen. Vorzugsweise verwendet man die Hydroxylamin-O-sulfonsäure in einem Überschuß von 10 bis

20 Mol.%.

In einigen Fällen kann es von Nutzen sein, durch Zugabe einer Base die Reaktion zu beschleunigen. Beispielsweise kann man 0 bis 3 Äquivalente der Base, bezogen auf II, zusetzen.

Geeignete Basen sind z.B. Alkalihydroxide wie NaOH oder KOH, Ammoniumhydroxid, Erdalkalihydroxide wie Magnesiumhydroxid oder Calciumhydroxid, Alkalicarbonate oder Alkalihydrogencarbonate.

Die Umsetzung kann bei Normaldruck, über- oder Unterdruck, kontinuierlich oder diskontinuierlich nach den dafür üblichen Techniken durchgeführt werden.

Die Isolierung der Cyanocyclohexenonverbindung Ia aus dem rohen Reaktionsgemisch kann in üblicher Weise, z.B. durch Extraktion oder Filtration, erfolgen.

Neben dem erfindungsgemäßen Verfahren können die Cyclohexenonverbindungen Ia auch durch Umsetzung von II mit Hydroxylamin und anschließender Wassereliminierung hergestellt werden. Weiterhin können die Verbindungen Ia nach den üblichen Methoden, ausgehend von den entsprechenden Carbonsäuren bzw. -derivaten oder den entsprechenden Halogenverbindungen hergestellt werden (vgl. Houben-Weyl Bd. E5/II, S. 1318ff).

Cyclohexenonverbindungen der allgemeinen Formel I, mit A gleich N-$OR^2$, in der $R^1$ und $R^2$ die oben angegebene Bedeutung haben, können auf bekannte Weise aus den Verbindungen Ia hergestellt werden.

Zu diesem Zweck setzt man letztere mit einem entsprechenden Hydroxylamin

$H_2N$-$OR^2$ III,

bzw. vorteilhaft mit dem Ammoniumsalz von III in Gegenwart einer Base um.

Zweckmäßigerweise führt man die Umsetzung in heterogener Phase in einem Lösungsmittel, bei einer ausreichenden Temperatur unterhalb von etwa 80 °C bzw. dem Siedepunkt des Reaktionsgemisches durch.

Geeignete Basen sind z.B. Carbonate, Hydrogencarbonate, Acetate, Alkoholate, Hydroxide oder Oxide von Alkali- oder Erdalkalimetallen, insbesondere Natrium-, Kaliumhydroxid, Magnesium-, Calciumoxid. Außerdem können organische Basen wie Pyridin oder tertiäre Amine Verwendung finden. Die Base wird beispielsweise in einer Menge von 0,5 bis 2 Mol, bezogen auf die Ammioniumverbindung zugesetzt (vgl. DE-A 34 33 767).

Als Lösungsmittel sind beispielsweise Dimethylsulfoxid, Alkohole wie Methanol, Ethanol, Isopropanol, aromatische Kohlenwasserstoffe wie Benzol, Toluol, chlorierte Kohlenwasserstoffe wie Chloroform, Dichlorethan, aliphatische Kohlenwasserstoffe wie Hexan, Cyclohexan, Ester wie Essigsäureethylester, Ether wie Dioxan, Tetrahydrofuran geeignet.

Die Umsetzung ist nach wenigen Stunden beendet, das Zielprodukt kann z.B. durch Einengen der Mischung, Verteilung des Rückstandes in Methylenchlorid/Wasser und Abdestillieren des Lösungsmittels unter vermindertem Druck isoliert werden.

Bei der Verwendung der freie Hydroxylaminbase III, z.B. in Form einer wäßrigen Lösung, erhält man je nach verwendetem Lösungsmittel für den Ausgangsstoff Ia ein ein- oder zweiphasiges Reaktionsgemisch.

Geeignete Lösungsmittel für diese Umsetzung sind beispielsweise Methanol, Ethanol, Isopropanol, aromatische Kohlenwasserstoffe wie Benzol, Toluol, chlorierte Kohlenwasserstoffe wie Chloroform, Dichlorethan, aliphatische Kohlenwasserstoffe wie Hexan, Cyclohexan, Ester wie Essigsäureethylester, Ether wie Dioxan, Tetrahydrofuran, Nitrile wie Acetonitril.

Alkalimetallsalze der Verbindungen I mit A gleich Sauerstoff oder N-$OR^2$ können durch Behandeln der 3-Hydroxyverbindungen mit Natrium-oder Kaliumhydroxid oder -alkoholat in wäßriger Lösung oder in einem organischen Lösungsmittel wie Methanol, Ethanol, Aceton, Toluol erhalten werden.

Andere Metallsalze, z.B. die Mangan-, Kupfer-, Zink-, Eisen-, Calcium-, Magnesium- und Bariumsalze können aus den Natriumsalzen in üblicher Weise hergestellt werden, ebenso Ammonium- und Phosphonium-Sulfonium- oder Sulfoxoniumsalze mittels Ammoniak, Phosphonium-, Sulfonium- oder Sulfoxoniumhydroxiden. Die Ester können durch Umsetzung einer Verbindung der Formel I mit einem Säurechlorid in Gegenwart einer Base (z.B. Triethylamin) in einem inerten Lösungsmittel nach den üblichen Methoden hergestellt werden.

Cyclohexenonverbindungen der allgemeinen Formel I, mit A gleich $NR^3$, in der $R^1$ und $R^3$ die oben angegebene Bedeutung haben, können auf bekannte Weise aus den Verbindungen der allgemeinen Formel Ia hergestellt werden.

Zu diesem Zweck setzt man die Cyclohexenonverbindung mit einem Amin $H_2NR^3$ um. Zweckmäßig führt man die Umsetzung in homogener Phase in einem Lösungsmittel, bei einer ausreichenden Temperatur unterhalb von etwa 80 °C bzw. dem Siedepunkt des Reaktionsgemisches, durch. Als Lösungsmittel sind beispielsweise Dimethylsulfoxid, Alkohole wie Methanol, Ethanol, Isopropanol, aromatische Kohlenwasserstoffe wie Benzol, Toluol, chlorierte Kohlenwasserstoffe wie Chloroform, Dichlorethan, aliphatische Kohlenwasserstoffe wie Hexan, Cyclohexan, Ester wie Essigsäureethylester, Ether wie Dioxan, Tetrahydrofuran geeignet.

Die Umsetzung ist nach wenigen Stunden beendet, das Zielprodukt kann durch Einengen der Mischung, Verteilung des Rückstandes in Methylenchlorid/Wasser und Abdestillieren des Lösungsmittels unter vermindertem Druck isoliert werden.

Die folgenden Beispiele erläuten die Herstellung der erfindungsgemäßen Cyclohexenonverbindungen I.

Herstellvorschriften

Beispiel 1.1

3,5-Dioxo-4-acetyl-cyclohexancarbonsäurenitril

19,3 g (0,11 Mol) 5-Formyl-2-acetyl-cyclohexan-1,3-dion wurden in 200 ml destilliertem Wasser vorgelegt und bei Raumtemperatur mit 14,7 g (0,13 Mol) Hydroxylamin-O-sulfonsäure versetzt. Anschließend wurde 17 h bei Raumtemperatur nachgerührt. Der Niederschlag wurde abgesaugt, mit Wasser gewaschen und getrocknet. Es wurden 11,5 g (61 % d. Th.) 3,5-Dioxo4-acetyl-cyclohexancarbonsäurenitril erhalten (Fp. 66 bis 69 °C).

Beispiel 1.2

3,5-Dioxo-4-propionyl-cyclohexancarbonsäurenitril

30,7 g (0,16 Mol) 5-Formyl-2-propionylcyclohexan-1,3-dion wurden in 150 ml destilliertem Wasser vorgelegt und bei Raumtemperatur mit 21,0 g (0,19 Mol) Hydroxylamin-O-sulfonsäure versetzt. Anschließend wurde über Nacht bei Raumtemperatur nachgerührt. Der Niederschlag wurde abgesaugt, mit Wasser gewaschen und getrocknet. Es wurden 23,8 g (71 % d. Th.) 3,5-Dioxo-4-propionyl-cyclohexancarbonsäurenitril erhalten (Fp. 70 bis 74 °C).

Beispiel 1.5

3,5-Dioxo-4-palmitoyl-cyclohexancarbonsäurenitril

43 g (0,12 Mol) 5-Formyl-2-palmitoyl-cyclohexan-1,3-dion wurden in 150 ml destilliertem Wasser suspendiert. Hierzu gab man 15,7 g (0,14 Mol) Hydroxylamin-O-sulfonsäure, gelöst in 50 ml Wasser. Das heterogene Reaktionsgemisch wurde über Nacht bei Raumtemperatur gerührt. Der erhaltene Feststoff wurde abgesaugt, mit Wasser gewaschen und getrocknet. Es wurden 37,7 g (86 % d. Th.) 3,5-Dioxo-4-palmitoyl-cyclohexancarbonsäurenitril isoliert (Fp. 66 bis 69 °C).

Die in Tabelle 1 dargestellten Cyclohexenonverbindungen Ia lassen sich auf analogem Wege herstellen.

Tabelle 1: Cyclohexenonverbindungen Ia

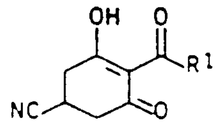

Ia

| Nr. | $R^1$ | phys. Daten<br>Fp [°C], $^1$H-NMR ($CDCl_3$, δ in ppm) |
|---|---|---|
| 1.1 | Methyl | 94 bis 97 |
| 1.2 | Ethyl | 70 bis 74 |
| 1.3 | Propyl | 47 bis 50 |
| 1.4 | Butyl | 58 bis 59 |
| 1.5 | Pentadecanyl | 66 bis 69 |
| 1.6 | Cyclopropyl | 1,2 (m,2H); 1,35 (m,2H); 3,0 (m,2H); 3,3 (m,1H); 3,55 (m,1H) |
| 1.7 | Cyclohexyl | |
| 1.8 | Phenyl | |
| 1.9 | 4-Chlor-2-nitrophenyl | 184-187 (Zers.) |

Beispiel 2.3

Herstellbeispiel für Oximether

3.1 g (0,016 Mol) 3,5-Dioxo-4-propionyl-cyclohexancarbonsäurenitril wurden in 50 ml trockenem Methanol gelöst und nacheinander mit 2,2 g (0,018 Mol) But-2-enyloxiaminhydrochlorid und 1,5 g (0,018 Mol) Natriumhydrogencarbonat versetzt. Anschließend wurde das Reaktionsgemisch 16 h bei Raumtemperatur gerührt. Zum Aufarbeiten wurde das Lösungsmittel im Vakuum abgezogen und der Rückstand mit Methylenchlorid/Methanol 98:2 über Kieselgel chromatographiert. Es wurden 2,2 g (52 % d. Th.) 3,5-Dioxo-4[1-(but-2-enyloximino)-propyl]-cyclohexancarbonsäurenitril isoliert.

Analog können die in Tabelle 2 aufgeführten Verbindungen Ib hergestellt werden.

Tabelle 2: Cyclohexenonverbindungen Ib

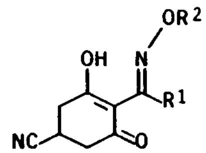

Ib

| Nr. | $R^1$ | $R^2$ | phys. Daten ($^1$H-NMR in $CDCl_3$, δ ppm) |
|---|---|---|---|
| 2.1 | Ethyl | Ethyl | 1.17 (t,3H); 1.34 (t,3H); 2.98 (q,2H); 3.15-3.33 (m,1H); 4.13 (q,2H); |
| 2.2 | Ethyl | Allyl | 1.14 (t,3H); 2.7-2.88 (m,4H); 2.96 (q,2H); 3.15-3.35 (m,1H); 4.56 (d,2H); |
| 2.3 | Ethyl | (E)-But-2-enyl | |
| 2.4 | Ethyl | (E)-3-Chlorprop-2-enyl | |
| 2.5 | Ethyl | (E)-4-(4-Fluorphenyl)-but-2-enyl | 1.15 (t,3H); 2.78 (m,4H); 2.95 (q,2H); 3.22 (m,1H); 3.41 (d,2H); 4.5 (d,2H); |
| 2.6 | Ethyl | 5-Chlorthenyl | |
| 2.7 | Methyl | (E)-3-Chlorprop-2-enyl | 2.35 (s,3H); 3.26 (m,1H); 4,54 (d,2H); 6.12 (m,1H); 6.38 (d,1H); |
| 2.8 | Methyl | (E)-But-2-enyl | 1.79 (d,3H); 2.47 (s,3H); 3.24 (m,1H); 4.48 (d,2H); 5.68 (m,1H); 5.9 (m,1H); |
| 2.9 | Propyl | Ethyl | 1.01 (t,3H); 1.35 (t,3H); 1.58 (dt,2H); 2.97 (t,2H); 3.25 (m,1H); 4.15 (q,2H); |
| 2.10 | Propyl | Allyl | 0.99 (t,3H); 1.58 (dt,2H); 2.93 (t,2H); 3.24 (m,1H); 4.46 (d,2H); |
| 2.11 | Propyl | (E)-3-Chlorprop-2-enyl | 0,97 (t,3H); 1.55 (dt,2H); 2.9 (t,2H); 3.26 (m,1H); 4.45 (d,2H); |
| 2.12 | Propyl | (E)-But-2-enyl | 0,96 (t,3H); 1.59 (dt,2H); 1.79 (d,3H); 2.94 (t,2H); 3.22 (m,1H); 4.47 (d,2H); |

| Nr. | $R^1$ | $R^2$ | phys. Daten ($^1H$-NMR in $CDCl_3$, δ ppm) |
|---|---|---|---|
| 2.13 | Propyl | 5-Chlorthenyl | 0,97 (t,3H); 1.54 (dt,2H); 2.86 (t,2H); 3.23 (m,1H); 5.1 (s,2H); 6.84 (d,1H); |
| 2.14 | Penta-decanyl | Ethyl | 0,88 (t,3H); 1.26 (m,31H); 2.8 (m,4H); 2.97 (d,2H); 3.23 (m,1H); 4.13 (q,2H); |
| 2.15 | Penta-decanyl | Allyl | 0,87 (t,3H); 1.26 (m,31H); 2.6-3.0 (m,6H); 3.23 (m,1H); 4.55 (d,2H); |
| 2.16 | Penta-decanyl | (E)-But-2-enyl | 0,88 (t,3H); 1.27 (m,31H); 1.8 (d,3H); 2.8 (m,4H); 2.96 (t,2H); 3.22 (m,1H); |
| 2.17 | Penta-decanyl | (E)-3-Chlorprop-2-enyl | 0,87 (t,3H); 1.25 (m,31H); 2.88 (m,6H); 3.25 (m,1H); 4.54 (d,2H); 6.12 (m,1H); |
| 2.18 | Penta-decanyl | Propargyl | 0,9 (t,3H); 1.24 (m,28H); 2.58 (t,1H); 2.9 (m,6H); 4.66 (d,2H); |
| 2.19 | Penta-decanyl | (E)-4-(4-Fluorphenyl)-but-2-enyl | 0,89 (t,3H); 1.26 (m,28H); 1.8 (d,3H); 2.8 (m,4H); 2.94 (t,2H); 3.22 (m,1H); |
| 2.20 | Penta-decanyl | 5-Chlorthenyl | 0,88 (t,3H); 1.24 (m,28H); 2.84 (m,6H); 3.21 (m,1H); 5.1 (s,2H); 6.85 (m,2H); |
| 2.21 | Butyl | Ethyl | |
| 2.22 | Methyl | Ethyl | 1,35 (t,3H); 3,45 (s,3H); 3,0 (m,1H); 4,15 (q,2H); |
| 2.23 | Methyl | 5-Chlorthenyl | 2,35 (s,3H); 3,25 (m,1H); 5,1 (s,2H); 6,85 (d,1H); 6,9 (d,1H); |
| 2.24 | 4-Chlor-2-nitro-phenyl | Ethyl | 0,85 u. 1,4 (t,3H); 7,1 u. 7,25 (d,H); 7,6 u. 7,7 (dd,1H); 8,15 (d,1H); |
| 2.25 | 4-Chlor-2-nitro-phenyl | Allyl | 7,15 u. 7,25 (d,1H); 7,6 u. 7,65 (d,1H); 8,1 (s,1H); |

Beispiel 3.3

1,2 g (0,006 Mol) 3,5-Dioxo-4-propionylcyclohexancarbonsäurenitril wurde in 40 ml trockenem Tetrahydrofuran gelöst und mit 0,4 g (0,006 Mol) Isopropylamin versetzt. Die Mischung wurde 16 h bei

Raumtemperatur gerührt, das Lösungsmittel im Vakuum abgezogen und der Rückstand mit Methylenchlorid/Methanol über etwas Kieselgel abgesaugt. Nach dem Abziehen des Lösungsmittels wurden 1,3 g (≙ 92 % d. Th.) 3,5-Dioxo-4-(1-isopropyliminopropyl)-cyclohexancarbonsäurenitril erhalten.

Analog können die in Tabelle 3 dargestellten Cyclohexenonverbindungen Ic hergestellt werden.

Tabelle 3: Cyclohexenonverbindungen Ic

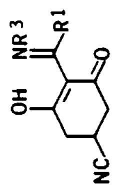

Ic

| Nr. | $R^1$ | $R^3$ | phys. Daten ($^1H$-NMR in $CDCl_3$, δ ppm) |
| --- | --- | --- | --- |
| 3.1 | Ethyl | Isobutyl | 1.06 (d,6H); 1.21 (t,3H); 1.99 (m,1H); 3.0 (q,2H); 3.2 (m,1H); 3.2 (t,2H); |
| 3.2 | Ethyl | s-Butyl | 0.98 (t,3H); 1.21 (t,3H); 1.3 (d,3H); 3.19 (m,1H); 3.8 (m,1H); |
| 3.3 | Ethyl | i-Propyl | 1.22 (t,3H); 1.35 (d,6H); 3.2 (q,2H); 3.19 (m, 1H); 4.03 (m,1H); |
| 3.4 | Ethyl | Benzyl | 1.2 (t,3H); 3.08 (q,2H); 3.2 (m,1H); 4.69 (d,2H); 7.35 (m,5H); |
| 3.5 | Methyl | Isobutyl | 1,05 (d,6H); 2,0 (m,1H); 2,55 (s,3H); 2, 75 (m,4H); |
| 3.6 | Cyclopropyl | Isopropyl | 1,3 (m,10H); 2,85 (m,4H); |
| 3.7 | Cyclopropyl | Isobutyl | 1,3 (m,10H); 2,85 (m,4H); 3,35 (m,1H); |

Die erfindungsgemäßen herbiziden Verbindungen Ib und wachstumsregulier enden Verbindungen Ia und Ic bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich nach den Verwendungszwecken; sie sollten in jedem Fall möglichst die feinste Verteilung der erfindungsgemäßen Wirkstoffe gewährleisten.

Die Verbindungen I eignen sich allgemein zur Herstellung von direkt versprühbaren Lösungen, Emulsionen, Pasten oder Öldispersionen. Als inerte Zusatzstoffe kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Toluol, Xylol, Paraffin, Tetrahydronaphthalin, alkylierte Naphthaline oder deren Derivate, Methanol, Ethanol, Propanol, Butanol, Cyclohexanol, Cyclohexanon, Chlorbenzol, Isophoron oder stark polare Lösungsmittel wie N,N-Dimethylformamid, Dimethylsulfoxid, N-Methylpyrrolidon oder Wasser in Betracht.

Wäßrige Anwendungsformen können aus Emulsionskonzentraten, Dispersionen, Pasten, netzbaren Pulvern oder wasserdispergierbaren Granulaten durch Zusatz von Wasser bereitet werden. Zur Herstellung von Emulsionen, Pasten oder Öldispersionen können die Substrate als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholothylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Formulierungen enthalten zwischen 0,1 und 95 Gew.%, vorzugsweise zwischen 0,5 und 90 Gew.%, Wirkstoff. Die Wirkstoffe werden dabei in einer Reinheit von 90 % bis 100 %, vorzugsweise 95 % bis 100 % (nach NMR-Spektrum) eingesetzt.

Die erfindungsgemäßen Verbindungen 1 können beispielsweise wie folgt formuliert werden:

I. Man vermischt 90 Gewichtsteile der Verbindung Nr. 1.1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung Nr. 1.3 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

III. 20 Gewichtsteile der Verbindung Nr. 1.3 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% ges Wirkstoffs enthält.

IV. 20 Gewichtsteile des Wirkstoffs Nr. 1.5 werden in einer Mischung gelöst, die aus 25 Gewichtsteilen Cyclohexanon, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch

Eingießen und feines Verteilen der Lösung in 100 000 Gewichtsteilen Wasser erhält man eine wäßrige Dispersion, die 0,02 Gew.% des Wirkstoffs enthält.

V. 20 Gewichtsteile des Wirkstoffs Nr. 2.1 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

VI. 3 Gewichtsteile des Wirkstoffs Nr. 2.2 werden mit 97 Gewichtsteilen feinteiligem Kaolin vermischt. Man erhält auf diese Weise ein Stäubemittel, das 3 Gew.% des Wirkstoffs enthält.

VII. 30 Gewichtsteile des Wirkstoffs Nr. 2.9 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Gewichtsteile des Wirkstoffs Nr. 2.12 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenil-Harnstoff-Formaldehyd-Kondensates und 68 Gewichtsteilen eines paraffinischen Mineralöls innig vermischt. Man erhält eine stabile ölige Dispersion.

IX. Man vermischt 90 Gewichtsteile der Verbindung Nr. 3.1 mit 10 Gewichtsteilen N-Methyl-α-pyrrolidon und erhält eine Lösung, die zur Anwendung in Form kleinster Tropfen geeignet ist.

X. 20 Gewichtsteile der Verbindung Nr. 3.2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

XI. 20 Gewichtsteile der Verbindung Nr. 3.4 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

XII. 20 Gewichtsteile des Wirkstoffs Nr. 1.1 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

XIII. 20 Gewichtsteile des Wirkstoffs Nr. 1.1 werden mit 3 Gewichtsteilen des Natriumsalzes der Diisobutylnaphthalina-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

XIV. 3 Gewichtsteile des Wirkstoffs Nr. 1.3 werden mit 97 Gewichtsteilen feinteiligem Kaolin vermischt. Man erhält auf diese Weise ein Stäubemittel, das 3 Gew.% des Wirkstoffs enthält.

XV. 30 Gewichtsteile des Wirkstoffs Nr. 2.7 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

XVI. 20 Gewichtsteile des Wirkstoffs Nr. 3.3 werden mit 2 Gewichtsteilen Calciumsalz der Dodecylbenzolsulfonsäure, 8 Gewichtsteilen Fettalkohol-polyglykolether, 2 Gewichtsteilen Natriumsalz eines Phenolsulfonsäure-Formaldehyd-Kondensates und 68 Gewichtsteilen eines paraffinischen Mineralöls innig vermischt. Man erhält eine stabile ölige Dispersion.

Die Applikation der herbiziden und wachstumsregulierenden Mittel bzw. der Wirkstoffe kann im Vorauflauf- oder im Nachauflaufverfahren erfolgen. Sind die Wirkstoffe für gewisse Kulturpflanzen weniger verträglich oder sollen diese Pflanzen möglichst unbeeinflußt weiterwachsen, so können Ausbringungstechniken angewandt werden, bei welchen die Mittel mit Hilfe der Spritzgeräte so gespritzt werden, daß die Blätter der Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff bei Anwendung als Herbizide betragen je nach Bekämpfungsziel, Jahreszeit, Zielpflanzen und Wachstumsstadium 0,001 bis 3,0, vorzugsweise 0,01 bis 2,0 kg/ha aktive Substanz (a.S.).

Die Verbindungen der Formel I können praktisch alle Entwicklungsstadien einer Pflanze verschiedenartig beeinflussen und werden deshalb auch als wachstumsregulatoren eingesetzt. Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren z.B. (Samenbeize, Bodenbehandlung, Blattapplikation oder Stamminjektion bei Bäumen)

d) von klimatischen Faktoren, z.B. Temperatur, Niederschlagsmenge, außerdem auch Tageslänge und Lichtintensität

e) von der Bodenbeschaffenheit (einschließlich Düngung),

f) von der Formulierung bzw. Anwendungsform des Wirkstoffs und schließlich

g) von den angewendeten Konzentrationen der aktiven Substanz.

Aus der Reihe der verschiedenartigen Anwendungsmöglichkeiten der erfindungsgemäßen Pflanzenwachstumsregulatoren der Formel I im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf; außerdem ist eine dunklere Blattfärbung zu beobachten.

Als vorteilhaft für die Praxis erweist eine verminderte Intensität des Wachstums von Gräsern an Straßenrändern, Hecken, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse ist auch die Erhöhung der Standfestigkeit von lageranfälligen Kulturen wie Getreide, Reis, Mais, Sonnenblumen und Soja. Die dabei verursachte Halmverkürzung und Halmverstärkung verringern oder beseitigen die Gefahr des "Lagerns" (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Bei Obst- und anderen Bäumen lassen sich mit den Wachstumsregulatoren Schnittkosten einsparen. Außerdem kann die Alternanz von Obstbäumen durch Wachstumsregulatoren gebrochen werden.

Durch Anwendung von Wachstumsregulatoren kann auch die seitliche Verzweigung der Pflanzen vermehrt oder gehemmt werden. Daran besteht Interesse, wenn z.B. bei Tabakpflanzen die Ausbildung von Seitentrieben (Geiztrieben) zugunsten des Blattwachstums gehemmt werden soll.

Mit Wachstumsregulatoren läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z.B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und - wegen der relativ geringen Blatt- bzw. Pflanzenmasse - dem Befall mit verschiedenen Krankheiten (z.B. Pilzkrankheit) vorgebeugt werden. Die Hemmung des vegetativen Wachstums ermöglicht außerdem bei vielen Kulturpflanzen eine dichtere Bepflanzung des Bodens, so daß ein Mehrertrag bezogen auf die Bodenfläche erzielt werden kann.

B. Mit den Wachstumsregulatoren lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt

in Getreide oder Soja zu steigern oder Gummibäume zum vermehrten Latexfluß zu stimulieren.
Dabei können die Verbindungen der Formel I Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Entwicklungsstadien als auch eine Beschleunigung bzw. Verzögerung der Reife der geernteten Pflanzenteile vor oder nach der Ernte erreichen.
Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus, das heißt die Förderung der Ausbildung von Trenngewebe zwischen Frucht- bzw. Blatt- und Sproßteil der Pflanze ist auch für ein gut kontrollierbares Entblättern von Nutzpflanzen wie beispielsweise Baumwolle wesentlich.

D. Mit Wachstumsregulatoren kann weiterhin der Wasserverbrauch von Pflanzen reduziert werden. Dies ist besonders wichtig für landwirtschaftliche Nutzflächen, die unter einem hohen Kostenaufwand künstlich bewässert werden müssen, z.B. in ariden oder semiariden Gebieten. Durch den Einsatz der erfindungsgemäßen Substanzen läßt sich die Intensität der Bewässerung reduzieren und damit eine kostengünstigere Bewirtschaftung durchführen. Unter dem Einfluß von Wachstumsregulatoren kommt es zu einer besseren Ausnutzung des vorhandenen Wassers, weil u.a.
- die Öffnungsweite der Stomata reduziert wird
- eine dickere Epidermis und Cuticula ausgebildet werden
- die Durchwurzelung des Bodens verbessert wird und
- das Mikroklima im Pflanzenbestand durch einen kompakteren Wuchs günstig beeinflußt wird.

E. Mit den Wachstumsregulatoren der Formel I kann ferner das Wachstum von unerwünschten Pflanzen eingeschränkt werden. In Ackerkulturen kann so die Konkurrenzfähigkeit von Unkräutern und Ungräsern gegenüber den Kulturpflanzen reduziert werden, ohne daß dabei eine direkte herbizide Wirkung vorliegt. Ebenso ist es z.B. in Obst- oder Rebenanlagen möglich, die Konkurrenzkraft des Unterwuchses zum Vorteil der Nutzpflanzen stark zu vermindern ohne dabei die entsprechenden Gräser und Kräuter abzutöten. Auf diese Weise wird eine geschlossene Bodenbedeckung und damit z.B. eine bessere Bodenbelebtheit und ein Schutz vor Erosion erzielt.

Die erfindungsgemäß zu verwendenden Wachstumsregulatoren der Formel I können den Kulturpflanzen sowohl vom Samen her (als Saatgutbeizmittel) als auch über den Boden, d.h. durch die Wurzel sowie - besonders bevorzugt - durch Spritzung über das Blatt zugeführt werden.

Infolge der hohen Pflanzenverträglichkeit kann die Aufwandmenge stark variiert werden.

In Anbetracht der Vielseitigkeit der Applikationsmethoden können die erfindungsgemäßen Verbindungen bzw. sie enthaltende Mittel in einer großen Zahl von Kulturpflanzen zur Beseitigung unerwünschter Pflanzen und zur Regulierung des Pflanzenwachstums eingesetzt werden.

Zur Verbreiterung des Wirkungsspektrums und zur Erzielung synergistischer Effekte können die erfindungsgemäßen Verbindungen I mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate, Chinolincarbonsäurederivate, Aryloxy-, Heteroaryloxyphenoxypropionsäuren sowie deren Salze, Ester und Amide und andere in Betracht.

Außerdem kann es von Nutzen sein, die Verbindungen I allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien.

Als weitere Wachstumsregulatoren kommen unter anderem in Betracht: Bestimmte quaternäre Ammoniumverbindungen, Triazolyle, Imidazole, Norbornanodiazetine, Imidazole, 4-Pyridine, Ethephon, Abscisinsäure und davon abgeleitete Strukturen.

Von Interesse ist ferner die Mischbarkeit mit Mineralsalzlösungen, welche zur Behebung von Ernährungs- und Spurenelementmängeln eingesetzt werden. Es können auch nichtphytotoxische Öle und Ölkonzentrate zugesetzt werden.

## Anwendungsbeispiele

Die herbizide Wirkung der Cyclohexenonverbindungen der Formel I ließ sich durch Gewächshausversuche zeigen:

Als Kulturgefäße dienten Plastikblumentöpfe mit lehmigem Sand mit etwa 3,0 % Humus als Substrat. Die Samen der Testpflanzen wurden nach Arten getrennt eingesät.

Bei Vorauflaufbehandlung wurden die in Wasser suspendierten oder emulgierten Wirkstoffe direkt nach Einsaat mittels fein verteilender Düsen aufgebracht. Die Gefäße wurden leicht beregnet, um Keimung und Wachstum zu fördern und anschließend mit durchsichtigen Plastikhauben abgedeckt, bis die Pflanzen angewachsen waren. Diese Abdeckung bewirkt ein gleichmäßiges Keimen der Testpflanzen, sofern dies nicht durch die Wirkstoffe beeinträchtigt wurde.

Zum Zwecke der Nachauflaufbehandlung wurden die Testpflanzen je nach Wuchsform erst bei einer Wuchshöhe von 3 bis 15 cm mit den in Wasser suspendierten oder emulgierten Wirkstoffen behandelt. Die Aufwandmenge für die Nachauflaufbehandlung betrug 0,25 kg/ha a.S.

Die Pflanzen wurden artenspezifisch bei Temperaturen von 10-25 °C bzw. 20-35 °C gehalten. Die Versuchsperiode erstreckte sich über 2 bis 4 Wochen. Während dieser Zeit wurden die Pflanzen gepflegt und ihre Reaktion auf die einzelnen Behandlungen wurde ausgewertet.

Bewertet wurde nach einer Skala von 0 bis 100. Dabei bedeutet 100 kein Aufgang der Pflanzen bzw. völlige Zerstörung zumindest der oberirdischen Teile und 0 keine Schädigung oder normaler Wachstumsverlauf.

Die in den Gewächshausversuchen verwendeten Pflanzen setzten sich aus folgenden Arten zusammen:

| Abkürz. | Lateinischer Name | Deutscher Name | Englischer Name |
|---|---|---|---|
| AVEFA | Avena fatua | Flughafer | wild oats |
| ECHCG | Echinochloa crus-galli | Hühnerhirse | barnyardgrass |
| LOLMU | Lolium multiforum | Ital. Raygras | annual ryegrass |
| MEDSA | Medicago sativa | Luzerne | alfalfa |
| SETIT | Setaria italica | Ital. Raygras | italian ryegrass |

Mit 0,25 kg/ha a.S. im Nachlaufverfahren eingesetzt, lassen sich mit dem Beispiel 2.4 unerwünschte grasartige Pflanzen sehr gut bekämpfen. Luzerne als breitblättrige Kulturpflanze erleidet keinerlei Schädigung.

In Anbetracht des erfaßbaren Wirkungsspektrums zur Unkrautbekämpfung, der Verträglichkeit für Kulturpflanzen oder der unerwünschten Beeinflussung des Wachstums derselben sowie angesichts der Vielfalt der Applikationsmethoden können die Cyclohexenonderivate der Formel I in einer großen Zahl von Kulturpflanzen eingesetzt werden. In Betracht kommen beispielsweise folgende Kulturen:

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rüben |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |
| Daucus carota | Möhre |
| Elaeis guineensis | Ölpalme |
| Fragaria vesca | Erdbeere |
| Glycine max | Sojabohne |
| Gossypium hirsutum (Gossypium arboreum, Gossypium herbaceum,Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersicon lycopersicum | Tomate |

| Botanischer Name | Deutscher Name |
|---|---|
| Malus spp. | Apfel |
| Manihot esculenta | Maniok |
| Medicago sativa | Luzerne |
| Mentha piperita | Pfefferminze |
| Musa spp. | Obst- und Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium corymbosum | Kulturheidelbeere |
| Vaccinium vitis-idaea | Preiselbeere |
| Vicia faba | Pferdebohnen |
| Vigna sinensis (V. unguiculata) | Kuhbohne |
| Vitis vinifera | Weinrebe |
| Zea mays | Mais |

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen (ca. 12,5 cm Durchmesser; Volumen ca. 500 ml) angezogen.

Im Vorauflaufverfahren wurden die Testsubstanzen in wäßriger Aufbereitung am Tage der Einsaat auf das Saatbett gegossen.

Im Nachauflaufverfahren wurden die zu prüfenden Substanzen in wäßriger Aufbereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleichssubstanz diente 2-Chlorethyltrimethylammoniumchlorid (CCC).

Gleichlaufend zur Reduzierung des Längenwachstums stieg die Farbintensität der Blätter an. Der erhöhte Chlorophyllgehalt läßt eine ebenfalls erhöhte Photosyntheserate und damit eine erhöhte Ertragsbildung erwarten.

Die Einzeldaten sind den folgenden Tabellen zu entnehmen; wobei die Konzentration in mg Wirkstoff pro Gefäß (WS/Gef.) angegeben ist:

Tabelle 1: Sommerweizen, Sorte "Ralle"

Vorauflaufbodenbehandlung

| Nr. d. chem. Beispiele | Konzentration mg WS/Gef. | relative Wuchshöhe |
|---|---|---|
| unbehandelt | - | 100 |
| CCC | 6 | 70,8 |
| 1.2 | 6 | 54,9 |

Tabelle 2: Sommerweizen, Sorte "Ralle"

Nachauflaufblattbehandlung

| Nr. d. chem. Beispiele | Konzentration mg WS/Gef. | relative Wuchshöhe |
|---|---|---|
| unbehandelt | - | 100 |
| CCC | 6 | 83,0 |
| 1.2 | 6 | 64,3 |

Tabelle 3: Sommergerste, Sorte "Aramir"

Vorauflaufbodenbehandlung

| Nr. d. chem. Beispiele | Konzentration mg WS/Gef. | relative Wuchshöhe |
|---|---|---|
| unbehandelt | - | 100 |
| CCC | 6 | 78,8 |
| 1.2 | 6 | 60,6 |

Tabelle 4: Sommergerste, Sorte "Aramir"

Nachauflaufblattbehandlung

| Nr. d. chem. Beispiele | Konzentration mg WS/Gef. | relative Wuchshöhe |
|---|---|---|
| unbehandelt | - | 100 |
| CCC | 6 | 91,6 |
| 1.2 | 6 | 58,5 |

Tabelle 5: Reis, Sorte "Bahia"

Nachauflaufbodenbehandlung

| Nr. d. chem. Beispiele | Konzentration mg WS/Gef. | relative Wuchshöhe |
|---|---|---|
| unbehandelt | - | 100 |
| CCC | 1,5 | 100,0 |
| | 6 | 99,6 |
| 1.2 | 1,5 | 56,9 |
| | 6 | 40,9 |
| 1.3 | 1,5 | 69,3 |
| | 6 | 46,2 |
| 3.1 | 1,5 | 94,2 |
| | 6 | 92,5 |
| 3.2 | 1,5 | 96,0 |
| | 6 | 90,7 |
| 3.3 | 1,5 | 94,2 |
| | 6 | 81,8 |
| 3.4 | 1,5 | 100,0 |
| | 6 | 88,9 |

Tabelle 6: Reis, Sorte "Bahia"

Nachauflaufblattbehandlung

| Nr. d. chem. Beispiele | Konzentration mg WS/Gef. | relative Wuchshöhe |
|---|---|---|
| unbehandelt | - | 100 |
| CCC | 1,5 | 98,1 |
| 1.2 | 1,5 | 53,5 |
| 1.3 | 1,5 | 53,5 |
| 3.1 | 1,5 | 72,9 |
| 3.2 | 1,5 | 74,4 |
| 3.3 | 1,5 | 68,4 |
| 3.4 | 1,5 | 66,9 |

Tabelle 7: Sonnenblume, Sorte "Spanners Allzweck"

Nachauflaufblattbehandlung

| Nr. d. chem. Beispiele | Konzentration mg WS/Gef. | relative Wuchshöhe |
|---|---|---|
| unbehandelt | - | 100 |
| CCC | 1,5 | 89,2 |
| 1.2 | 1,5 | 73,9 |
| 1.3 | 1,5 | 72,5 |

Tabelle 8: Sommerraps, Sorte "Petranova"

Nachauflaufblattbehandlung

| Nr. d. chem. Beispiele | Konzentration mg WS/Gef. | relative Wuchshöhe |
|---|---|---|
| unbehandelt | - | 100 |
| CCC | 1,5 | 95,9 |
| 1.2 | 1,5 | 70,2 |
| 1.3 | 1,5 | 83,9 |
| 3.1 | 1,5 | 82,2 |
| 3.2 | 1,5 | 82,2 |
| 3.3 | 1,5 | 78,8 |
| 3.4 | 1,5 | 83,9 |

## Patentansprüche

**1.** Cyclohexenonverbindungen der Formel I

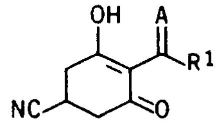

I,

in der die Substituenten die folgende Bedeutung haben:
$R^1$ $C_1$-$C_{20}$-Alkyl, $C_2$-$C_{20}$-Alkenyl, $C_2$-$C_{20}$-Alkinyl, $C_3$-$C_6$-Cycloalkyl, jeweils ggf. substituiert durch einen oder mehrere Halogensubstituenten, $C_2$-$C_6$-Alkoxyalkyl, $C_2$-$c_6$-Alkylthioalkyl, Benzyl oder Phenyl, wobei die aromatischen Kerne jeweils unsubstituiert oder ein- bis dreifach durch Halogen, Cyano, $C_1$-$C_4$-Alkyl, $C_1$-$C_4$-Alkoxy, $C_1$-$C_4$-Halogenalkyl, oder Nitro substituiert sind;
A Sauerstoff, $NOR^2$ oder $NR^3$, wobei
$R^2$ $C_1$-$C_4$-Alkyl, $C_3$-$C_4$-Alkenyl, $C_3$-$C_4$-Alkinyl, jeweils gegebenenfalls substituiert durch einen oder mehrere Substituenten aus der Gruppe Halogen, $C_1$-$C_4$-Alkoxy, Phenyl oder Heteroaryl mit 5 bis 6 Ringgliedern und 1 bis 2 Heteroatomen wie Stickstoff, Sauerstoff oder Schwefel, wobei die beiden

letztgenannten Reste unsubstituiert oder ein- bis dreifach durch Halogen, $C_1$-$C_4$-Alkyl, $C_1$-$C_4$-Alkoxy, $C_1$-$C_4$-Halogenalkyl oder Nitro substituiert sind,
$R^3$ Wasserstoff, $C_1$-$C_6$-Alkyl, $C_1$-$C_4$-Hydroxyalkyl, $C_2$-$C_4$-Alkoxyalkyl, $C_2$-$C_4$-Alkylthioalkyl, Phenyl oder Benzyl, wobei die beiden letztgenannten Reste unsubstituiert oder ein- bis dreifach durch Halogen, $C_1$-$C_4$-Alkyl, $C_1$-$C_4$-Alkoxy, $C_1$-$C_4$-Halogenalkyl oder Nitro substituiert sind,
sowie deren landwirtschaftlich brauchbaren Salze und Veresterungsprodukte mit Carbonsäuren, Sulfon- oder Phosphonsäuren.

**2.** Cyclohexenonverbindungen der Formel I gemäß Anspruch 1, in der die Substituenten die folgende Bedeutung haben:
$R^1$ Methyl, Ethyl, n-Propyl, n-Butyl, Cyclopropyl,
A Sauerstoff oder $NR^3$
$R^3$ Wasserstoff, Methyl, Ethyl, n-Propyl, i-Propyl, n-Butyl, s-Butyl, i-Butyl, n-Hexyl, Allyl, 2-Methoxyethyl, Benzyl, Phenyl.

**3.** Cyclohexenonverbindungen der Formel I gemäß Anspruch 1, in der die Substituenten die folgende Bedeutung haben:
$R^1$ Methyl, Ethyl, n-Propyl, n-Butyl, Cyclopropyl,
A N-$OR^2$,
$R^2$ Ethyl, Allyl, (E)-But-2-en-1-yl, Propargyl, But-2-in-1-yl, (E)-3-Chlorprop-2-en-1-yl, 4-(4-Fluorphenyl)-but-3-en-1-yl,4-(4-Chlorphenyl)-but-2-en-1-yl, 4-(4-Fluorphenyl)-but-3-en-1-yl, 4-(4-Chlorphenyl)-but-3-en-1-yl, 5-Chlorthenyl.

**4.** Verfahren zur Herstellung von Cyclohexenonverbindungen der Formel Ia

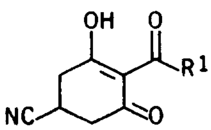

Ia,

in der $R^1$ die in Anspruch 1 genannte Bedeutung hat, dadurch gekennzeichnet, daß man ein Cyclohexenon der Formel II

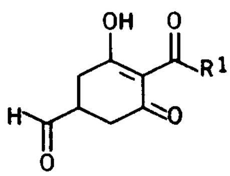

II,

in der $R^1$ die oben genannte Bedeutung hat, mit Hydroxylamin-O-sulfonsäure in einem inerten Lösungsmittel bei Temperaturen von 0 bis 150 °C umsetzt.

**5.** Verfahren nach Anspruch 4, dadurch gekennzeichnet, daß man die Umsetzung in homogener oder heterogener wäßriger Phase bei einer Temperatur von 20 bis 80 °C und einem pH-Bereich von 1 bis 9 durchführt.

**6.** Verfahren zur Herstellung von Cyclohexenonverbindungen der Formel Ib oder Ic

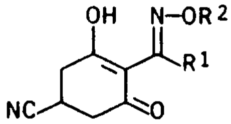

Ib,

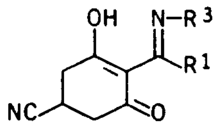

Ic,

in denen die Reste $R^1$, $R^2$ und $R^3$ die in Anspruch 1 genannte Bedeutung haben, dadurch gekennzeichnet, daß man eine Cyclohexenonverbindung der Formel Ia

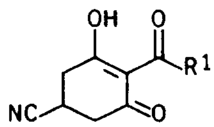

Ia

mit einem Hydroxylamin der Formel III

$H_2N\text{-}OR^2$ III

im Fall der Herstellung von Ib oder einem Amin der Formel IV

$H_2N\text{-}R^3$ IV

im Fall der Herstellung von Ic, wobei die Reste $R^2$ und $R^3$ die in Anspruch 1 genannte Bedeutung haben, in an sich bekannter Weise umsetzt.

7. Herbizides Mittel, enthaltend mindestens eine Cyclohexenonverbindung der Formel I gemäß Anspruch 1, in der A für den Rest $NOR^2$ steht, sowie übliche inerte Zusatzstoffe.

8. Verfahren zur Bekämpfung von unerwünschtem Pflanzenwuchs, dadurch gekennzeichnet, daß man die Pflanzen und/oder die von den Pflanzen freizuhaltende Fläche mit einer herbizid wirksamen Menge einer Cyclohexenonverbindung der Formel I gemäß Anspruch 1, in der A für den Rest $NOR^2$ steht, behandelt.

9. Mittel zur Regulierung des Pflanzenwachstums, enthaltend eine Cyclohexenonverbindung der Formel I gemäß Anspruch 1, in der A für Sauerstoff oder einen Rest $NR^3$ steht, sowie übliche inerte Zusatzstoffe.

10. Verfahren zur Regulierung des Pflanzenwachstums dadurch gekennzeichnet, daß man eine bioregulatorisch wirksame Menge einer Cyclohexenonverbindung der Formel I gemäß Anspruch 1, in der A für Sauerstoff oder einen Rest $NR^3$ steht, auf Pflanzen und/oder deren Lebensraum einwirken läßt.

## Claims

1. A cyclohexenone compound of the formula I

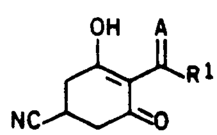

I,

where
$R^1$ is $C_1$-$C_{20}$-alkyl, $C_2$-$C_{20}$-alkenyl, $C_2$-$C_{20}$-alkynyl or $C_3$-$C_6$-cycloalkyl, each of which is unsubstituted or substituted by one or more halogen substituents, $C_2$-$C_6$-alkoxyalkyl,$C_2$-$C_6$-alkylthioalkyl, benzyl or phenyl, and the aromatic nuclei are each unsubstituted or monosubstituted to trisubstituted by halogen, cyano, $C_1$-$C_4$-alkyl, $C_1$-$C_4$-alkoxy, $C_1$-$C_4$-haloalkyl or nitro,
A is oxygen, $NOR^2$ or $NR^3$,
$R^2$ is $C_1$-$C_4$-alkyl, $C_3$- or $C_4$-alkenyl or $C_3$-or $C_4$-alkynyl, each of which is unsubstituted or substituted by one or more substituents from the group consisting of halogen, $C_1$-$C_4$-alkoxy, phenyl and hetaryl having 5 or 6 ring members and 1 or 2 hetero atoms, such as nitrogen, oxygen or sulfur, and the two last-mentioned radicals are unsubstituted or monosubstituted to trisubstituted by halogen, $C_1$-$C_4$-alkyl, $C_1$-$C_4$-alkoxy, $C_1$-$C_4$-haloalkyl or nitro,
$R^3$ is hydrogen, $C_1$-$C_6$-alkyl, $C_1$-$C_4$-hydroxyalkyl, $C_2$-$C_4$-alkoxyalkyl, $C_2$-$C_4$-alkylthioalkyl, phenyl or benzyl, and the two last-mentioned radicals are unsubstituted or monosubstituted to trisubstituted by

halogen, $C_1$-$C_4$-alkyl, $C_1$-$C_4$-alkoxy, $C_1$-$C_4$-haloalkyl or nitro,
and their agriculturally useful salts and esterification products with carboxylic acids, sulfonic acids or phosphonic acids.

**2.** A cyclohexenone compound of the formula I as claimed in claim 1, where $R^1$ is methyl, ethyl, n-propyl, n-butyl or cyclopropyl, A is oxygen or $NR^3$, and $R^3$ is hydrogen, methyl, ethyl, n-propyl, isopropyl, n-butyl, s-butyl, isobutyl, n-hexyl, allyl, 2-methoxyethyl, benzyl or phenyl.

**3.** A cyclohexenone compound of the formula I as claimed in claim 1, where $R^1$ is methyl, ethyl, n-propyl, n-butyl or cyclopropyl, A is $N$-$OR^2$, and $R^3$ is ethyl, allyl, (E)-but-2-en-1-yl, propargyl, but-2-yn-1-yl, (E)-3-chloroprop-2-en-1-yl, 4-(4-fluorophenyl)-but-3-en-1-yl, 4-(4-chlorophenyl)-but-2-en-1-yl, 4-(4-fluorophenyl)-but-3-en-1-yl, 4-(4-chlorophenyl)-but-3-en-1-yl or 5-chlorothenyl.

**4.** A process for the preparation of a cyclohexenone compound of the formula Ia

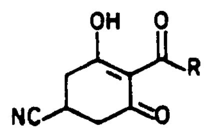

Ia,

where $R^1$ has the meanings given in claim 1, which comprises reacting cyclohexenone of the formula II

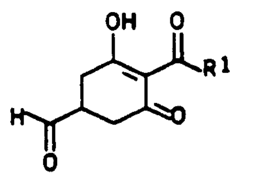

II,

where $R^1$ has the abovementioned meanings, with hydroxylamine-O-sulfonic acid in an inert solvent at from 0 to 150 °C.

**5.** A process as claimed in claim 4, wherein the reaction is carried out in the homogeneous or heterogeneous aqueous phase at from 20 to 80 °C and at a pH of from 1 to 9.

**6.** A process for the preparation of a cyclohexenone compound of the formula Ib or Ic

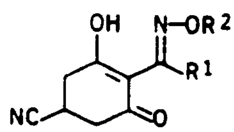

Ib,

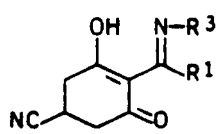

Ic,

where $R^1$, $R^2$ and $R^3$ have the meanings given in claim 1, which comprises reacting a cyclohexenone compound of the formula Ia

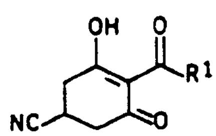

Ia

in a conventional manner with a hydroxylamine of the formula III

$H_2N$-$OR^2$ III

in the case of the preparation of Ib, or with an amine of the formula IV

$H_2N\text{-}R^3$ IV

in the case of the preparation of Ic, where $R^2$ and $R^3$ have the meanings given in claim 1.

**7.** A herbicide containing at least one cyclohexenone compound of the formula I as claimed in claim 1, where A is $NOR^2$, and conventional inert additives.

**8.** A method for controlling undesirable plant growth, wherein the plants and/or the area to be kept free from the plants are (is) treated with a herbicidally effective amount of a cyclohexenone compound of the formula I as claimed in claim 1, where A is $NOR^2$.

**9.** A plant growth regulator containing a cyclohexenone compound of the formula I as claimed in claim 1, where A is oxygen or $NR^3$, and conventional inert additives.

**10.** A method for regulating plant growth, wherein a bioregulatory amount of a cyclohexenone compound of the formula I as claimed in claim 1, where A is oxygen or $NR^3$, is allowed to act on plants and/or their habitat.

**Revendications**

**1.** Dérivés de cyclohexenone de la formule I

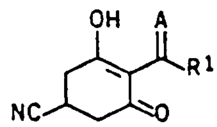

I,

dans laquelle les substituants ont la signification suivante :

- R1, alkyle en C1-C20, alcényle en C2-C20, alkinyle en C2-C20, cycloalkyle en C3-C6 chacun éventuellement substitué par un ou plusieurs substituants halogène, alcoxyalkyle en C2-C6, alkylthioalkyle en C2-C6, benzyle ou phényle les noyaux aromatiques étant chacun non substitués ou substitués une à trois fois par halogène, cyano, alkyle en C1-C4, alcoxy en C1-C4, halogènalkyle en C1-C4, ou nitro
- A, oxygène, NOR2 ou NR3,
- R2, alkyle en C1-C4, alcényle en C3-C4, alkinyle en C3-C4 chacun éventuellement substitue par un ou plusieurs substituants des groupes halogène, alcoxy en C1-C4 phényle ou hétéroaryle à 5 à 6 chaînons et 1 à 2 hétéroatomes tels que azote, oxygène ou soufre, les deux restes indiqués en dernier n'étant pas substitués ou substitués une à trois fois par halogène, alkyle en C1-C4, alcoxy en C1-C4, halogénoalkyle en C1-C4 ou nitro
- R3, hydrogène alkyle en C1-C6, hydroxyalkyle en C1-C4, alcoxyalkyle en C2-C4, alkylthioalkyle en C2-C4, phényle ou benzyle, les deux restes indiqués en derniers n'étant pas substitués ou substitués une à trois fois par halogène, alkyle en C1-C4, alcoxy en C1-C4, halogènalkyle en C1-C4 ou nitro

ainsi que leurs sels utilisables en agriculture et les produits d'estérification avec des acides carboxyliques, sulfoniques ou phosphoniques.

**2.** Dérivés de cyclohexenone de la formule I selon la revendication 1 dans laquelle les substituants ont les significations suivantes :

- R1, méthyle, éthyle, n-propyle, n-butyle, cyclopropyle
- A, oxygène ou NR3
- R3, hydrogène, méthyle, éthyle, n-propyle, i-propyle, n-butyle, s-butyle, i-butyle, n-hexyle, allyle, 2-méthoxyéthyle, benzyle, phényle.

**3.** Dérivés de cyclohexénone de la formule I dans laquelle les substituants ont la significations suivante :

R1, méthyle, éthyle, n-propyle, n-butyle, cyclopropyle

A, N-OR$^2$

R2, éthyle, allyle, (E)-but-2-en-1-yle, propargyle, but-2-en-1-yle, (E)-3-chloroprop-2-en-1-yle, 4-(4-fluorophényle)-but-3-en-1-yle, 4-(4-chlorophényle)-but-2-en-1-yle, 4-(4-fluorophényle)-but-3-en-1-yle, 4-(4-chlorophényle)-but-3-en-1-yle, 5-chlorothényle.

**4.** Procédé de préparation de dérivés de cyclohexenone de formule Ia

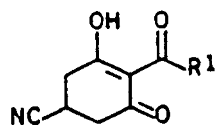

Ia,

dans laquelle R1 à la signification indiquée dans le revendication 1, caractérisé par le fait que l'on fait réagir une cyclohexenone de formule II

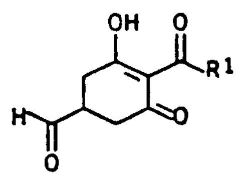

II,

dans laquelle R1 a la signification sus-indiquée avec l'acide hydroxylamine-O-sulfonique dans un solvant inerte, à des températures de 0 à 150 ° C.

**5.** Procédé selon la revendication 4, caractérisé par le fait que l'on effectue la réaction en phase homogène ou hétérogène aqueuse, à une température de 20 à 80 ° C dans un domaine de pH de 1 à 9.

**6.** Procédé de préparation de dérivés de cyclohexenone de formule Ib ou Ic

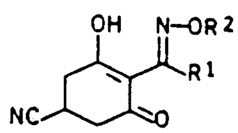

Ib,

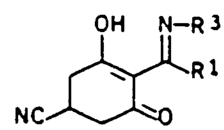

Ic,

dans laquelle les restes R1, R2 et R3 ont la signification indiquée dans le revendication 1, caractérisé par le fait que l'on fait réagir de manière connue en soi un dérivé de cyclohexenone de formule Ia

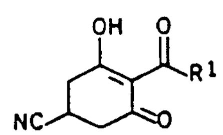

Ia

avec une hydroxylamine de formule III

$H_2N$-OR$^2$ III

dans le cas de la préparation de Ib, ou avec une amine de formule IV

$H_2N-R^3$ IV

dans le cas de préparation de Ic, les restes R2 et R3 ayant la signification indiquée dans la revendication 1.

**7.** Agent herbicide, contenant au moins un dérivé de cyclohexenone de formule I selon la revendication 1, dans laquelle A est mis pour le reste $NOR^2$, ainsi que des additifs inertes usuels.

**8.** Procédé pour lutter contre la croissance de plantes indésirables, caractérisé par le fait que l'on traite les plantes et/ou les surfaces à maintenir exemptes de plantes, avec une quantité à activité herbicide d'un dérivé de cyclohexenone de formule I selon la revendication 1 dans laquelle A est mis pour le reste $NOR^2$.

**9.** Moyen de régulation de la croissance des plantes contenant un dérivé de cyclohexenone de formule I selon la revendication 1 dans laquelle A est mis pour oxygène ou un reste $NR^3$ ainsi que des additifs inertes usuels.

**10.** Procédé de régulation de la croissance des plantes, caractérisé par le fait que l'on fait agir sur les plantes et/ou leur biotope, une quantité à activité biorégulatrice d'un dérivé de cyclohexenone de formule I selon la revendication 1 dans laquelle A est mis pour oxygène ou un reste $NR^3$.